# EUROPÄISCHE PATENTSCHRIFT

(11) **EP 0 867 157 B1**
(45) Veröffentlichungstag und Bekanntmachung des Hinweises auf die Patenterteilung: **31.07.2002**
(21) Anmeldenummer: 98102733.7
(22) Anmeldetag: 17.02.1998
(51) Int. Cl.: A61F 2/30

(54) **Gelenkendoprothese**
Joint endoprosthesis
Endoprothèse d'articulation

(30) Priorität: 26.03.1997 DE 29705499 U
(43) Veröffentlichungstag der Anmeldung: 30.09.1998
(73) Patentinhaber: Waldemar Link (GmbH & Co.), 22339 Hamburg (DE)
(72) Erfinder: Keller, Arnold, 23863 Kayhude (DE)
(74) Vertreter: Glawe, Delfs, Moll & Partner

(56) Entgegenhaltungen:
- EP-A- 0 158 014
- EP-A- 0 649 639
- EP-A- 0 654 255
- CH-A- 640 132
- FR-A- 2 602 672

## Beschreibung

Ansatzteile von Endoprothesen, die während der Implantation angesetzt werden, oder bei einer Reoperation entfernt werden, sollten auf möglichst einfache Weise befestigt und gesichert werden, damit dieser Vorgang möglichst wenig Aufmerksamkeit und handwerkliche Geschicklichkeit in Anspruch nimmt. *Bei einer Hüftgelenkendoprothese, die eine im Beckenknochen zu implantierende Außenschale sowie eine Innenschale aufweist, ist es bekannt (EP-A-0 654 255), zur Befestigung der Innenschale in der Außenschale eine Schraube vorzusehen. Deren Kopf wirkt mit Keilflächen der Außenschale und Innenschale zusammen. Zur Fixierung zieht der Operateur die Schraube soweit an, bis sie mit ihrem* Kopf *zwischen Innenschale und Außenschale verkeilt ist; damit ist die Innenschale in der Außenschale gehalten. Nachteilig ist die verhältnismäßig umständliche Handhabung, da die Schraube vor dem Einsetzen der Innenschale in ihrer Aufnahmebohrung einzusetzen und ein kleines Stück zu verdrehen ist. Die erfordert Geschick und zusätzliche Aufmerksamkeit. Ferner ist es* bekannt, für die Verbindung einer Endoprothese mit einem Ansatzteil zusammenwirkende Führungsmittel an der Prothese und dem Ansatzteil vorzusehen, die leicht ineinandergeschoben werden können und einen wesentlichen Teil der Kraftübertragung bei der Prothesenbenutzung übernehmen. Die Sicherung des Ansatzteils kann sich dann darauf beschränken, seine Relativbewegung entlang der Führung zu verhindern. Bekannt ist es, dafür eine Schraube zu verwenden, die im Bereich von einander überlappenden Flächenanteilen der Prothese und des Ansatzteils vorgesehen ist. Einer dieser Flächenanteile enthält eine die Schraube aufnehmende Gewindebohrung. Der andere Flächenanteil enthält eine Ausnehmung, die einen Teil der Schraube aufnimmt, wenn diese in ihrer sichernden Endstellung ist. Ein Beispiel dafür bildet eine bekannte Hüftgelenk-Endoprothese (Prospekt der Fa. Waldemar Link (GmbH & Co): "Rippensystem"), deren in den Oberschenkelknochen einzusenkender Schaft in einer Halsauflage endet, die größtenteils längs einer am Schaft vorgesehenen Schiebeführung ansetzbar bzw. entfernbar ist. Ein parallel zu der Halsauflage sich erstreckender Ansatz am Schaft enthält eine Bohrung, durch die eine Schraube in eine in der Halsauflage vorgesehene Gewindebohrung eingesetzt wird. Dies erfordert Geschick.

Der Erfindung liegt daher die Aufgabe zugrunde, eine Gelenkendoprothese der im Oberbegriff des Anspruchs 1 genannten Art zu schaffen, bei der die Sicherung des Ansatzstücks weniger Aufmerksamkeit und Geschick erfordert. Die Lösung liegt in den Merkmalen des Anspruchs 1 und vorzugsweise denjenigen der Unteransprüche.

Vor dem Anbringen des Ansatzstücks wird dafür gesorgt, daß die Schraube bis zum Erreichen ihrer Endstellung in die Gewindebohrung des ersten Flächenanteils eingeschraubt ist. Sie befindet sich damit in sicherungsbereiter Stellung und braucht nicht lose in bestimmter Stellung gehalten zu werden. Auch ihre axiale Einstellung bedarf während der Operation keine Aufmerksamkeit, weil sie vorbereitend bis zum Anschlag eingedreht werden kann. Ein Irrtum ist dabei nicht möglich. Der Abschnitt reduzierter Dicke, der sich an dem Gewindeabschnitt anschließt, liegt dann in dem Bereich, in welchem der zweite Flächenanteil des Ansatzteils zu erwarten ist. Dieser weist an der entsprechenden Stelle einen Schlitz auf, der so angeordnet ist, daß er beim Einschieben des Ansatzteils in die Führung den Abschnitt reduzierter Dicke aufnimmt. Das verdickte Ende der Schraube liegt dann, gesehen vom ersten Flächenanteil aus, jenseits des zweiten Flächenanteils in einem gewissen Abstand davon, der die Verschiebung des Ansatzteils gestattet. Hat der Ansatzteil seine gewünschte Endstellung erreicht, wird die Schraube zurückgedreht, bis das verdickte Ende der Schraube an dem zweiten Flächenanteil, nämlich an den Kanten des Schlitzes, in Eingriff kommt und dadurch den Ansatzteil fixiert. Der Operateur braucht daher lediglich die Schraube aus ihrer einen Endstellung in die andere zu drehen. Dies erfordert keinerlei besondere Aufmerksamkeit. Es ist auch nur eine kurze Drehung der Schraube erforderlich, weil lediglich die Distanz von der einen in die andere Endstellung überwunden zu werden braucht, die sehr gering sein kann und mit dem Bruchteil einer Vollumdrehung der Schraube überwunden werden kann. Mehrmaliges Nachfassen des Schraubendrehers ist in der Regel nicht erforderlich.

Das Zusammenwirken des verdickten Schraubenendes mit den Rändern des im zweiten Flächenanteil vorgesehenen Schlitzes verlangt im allgemeinen keinen Formschluß für die Sicherungsfunktion. Die Pressung, die sich beim Andrehen der Schraube in ihre Endstellung ergibt, reicht aus. Jedoch kann gewünschtenfalls Formschluß vorgesehen werden, indem beispielsweise am geschlossenen Ende des Schlitzes eine die Verdickung wenigstens teilweise aufnehmende Vertiefung vorgesehen wird. Zusätzlich oder statt dessen kann die dem Schlitz zugewendete Seite des verdickten Schraubenendes konisch ausgebildet sein und dadurch bereits durch geringen Anzug der Schraube zu einer plastischen Verformung führen, die nicht nur den Ansatzteil sichert, sondern auch eine so hohe Reibung an der Schraube zur Folge hat, daß diese sich nicht zufällig aus ihrer Endstellung lösen kann.

Vorzugsweise wird die Gewindebohrung in demjenigen Flächenanteil vorgesehen, der leichter zugänglich ist, wobei der Schraubenkopf, der beispielsweise einen Sechskant zum Zusammenwirken mit dem Schraubendreher aufweist, dem Operateur zugewandt ist. Jedoch ist auch die umgekehrte Anordnung denkbar. Dann sollte das verdickte Ende der Schraube mit einer Ansatzformation, beispielsweise einem Sechskant, für das Drehwerkzeug versehen sein.

Die Erfindung wird im folgenden unter Bezugnahme auf die Zeichnung erläutert, die ein vorteilhaftes Ausführungsbeispiel veranschaulicht. Es zeigen:
- Fig. 1: die Draufsicht auf den oberen Bereich einer Oberschenkelhüftprothese mit einer aufzuschiebenden Halsauflage,
- Fig. 2: den Schraubenbereich derselben in vergrößerter Querschnittsdarstellung vor der Sicherung und
- Fig. 3: den Zustand nach Sicherung.

Die Hüftprothese, deren Hals 1 im Schnitt erkennbar ist, weist eine abnehmbare Halsauflage 2 auf, die auf einen Kragenteil 3 an der Prothese aufzuschieben ist. Dazu dienen an sich bekannte und daher im Detail nicht dargestellte Führungseinrichtungen, deren Kontur gestrichelt bei 4 angedeutet ist. Die Stellung der Halsauflage nach dem Aufschieben ist strichpunktiert links angedeutet. Zum Sichern der Halsauflage in der aufgeschobenen Stellung dient eine Schraube 5, deren Schaft von einem Schlitz 6 der Halsauflage aufgenommen wird. Die Schraube 5 sitzt in einem Flächenanteil 7 des Kragens 3. Der zugehörige Schlitz 6 ist in einem Flächenanteil 8 der Halsauflage angeordnet.

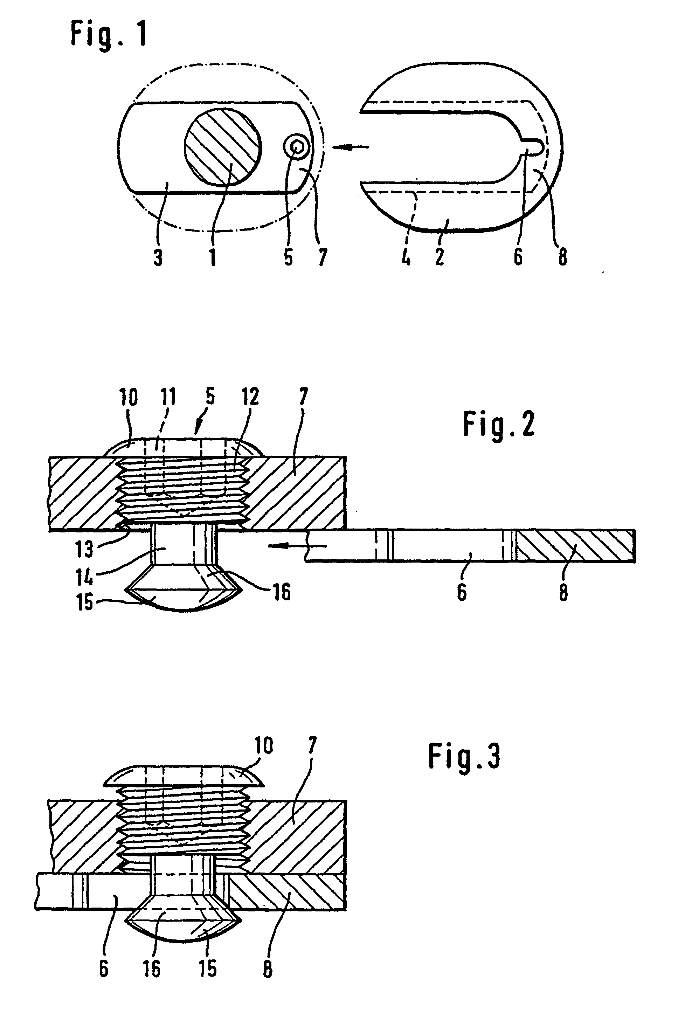

Diese Flächenanteile erkennt man in Fig. 2 im Zustand vor der Beendigung des Aufschiebens der Halsauflage. Der Schlitz 6 hat die Schraube 5 noch nicht erreicht.

Die Schraube 5 besteht aus einem Kopf 10 mit Innensechskant 11 für den Angriff eines Schraubendrehers, einem Gewinde 12, das in einer Gewindebohrung 13 des Flächenanteils 7 sitzt und dessen axiale Länge nicht größer als die Dicke des Flächenanteils 7 ist, einem Abschnitt reduzierter Dicke 14 und einem verdickten Ende 15, das dem Gewindeabschnitt eine konische Fläche 16 zukehrt. Die Schraube ist in dem sicherungsbereiten Zustand vollständig in den Flächenanteil 7 eingedreht, so daß der Kopf an dessen Oberfläche anliegt. In diesem Zustand ist die freie Länge des Abschnitts 14 reduzierter Dicke mindestens so groß wie die Dicke des Flächenanteils 8.

Nachdem der Flächenanteil 8 in seine Endstellung gelangt ist, in der sein Schlitz 6 den Abschnitt 14 reduzierter Dicke umgibt, wird die Schraube 5 so gedreht, daß ihr Kopf 10 sich von der Oberfläche des Flächenanteils 7 löst. Dabei nähert sich das verdickte Ende 15 der Schraube dem Flächenanteil 8, bis schließlich seine Konusfläche 16 die Ränder des Schlitzes 6 erfaßt. Wenn die Schraube in die Stellung, die in Fig. 3 dargestellt ist, hinreichend fest angedreht ist, sichert sie den Flächenanteil 8 bzw. die Halsauflage 2 in ihrer Funktionsstellung.

## Patentansprüche

1. Gelenkendoprothese mit einem Ansatzteil (2), das längs einer an der Endoprothese vorgesehenen Führung (3, 4) ansetzbar bzw. entfernbar ist und mit einer Schraube (5) zum Arretieren des Ansatzteils (2), *die einen Kopf (10) sowie einen Schaft mit einem dem Kopf (10) benachbarten Gewinde (12) und* die im Bereich von an der Prothese und am Ansatzteil (2) einander überlappenden Flächenanteilen (7, 8) angeordnet ist, von denen ein erster Flächenanteil (7) eine die Schraube (5) aufnehmende Gewindebohrung (13) enthält, **dadurch gekennzeichnet, daß** der Schraubenschaft *anschließend an den* Gewindeabschnitt (12), einen Abschnitt (14) reduzierter Dicke und daran anschließend ein verdicktes Ende (15) aufweist, wobei der Abschnitt (14) reduzierter Dicke im Bereich des zweiten Flächenanteils (8) liegt, der einen in Richtung der Führung (3, 4) verlaufenden, den Abschnitt (14) reduzierter Dicke aufnehmenden Schlitz (6) aufweist, dessen Weite geringer ist als die Dicke des verdickten Endes (15).

2. Prothese nach Anspruch 1, **dadurch gekennzeichnet, daß** der erste, die Gewindebohrung (13) enthaltende Flächenanteil (7) der leichter zugängliche ist.

3. Prothese nach Anspruch 1 oder 2, **dadurch gekennzeichnet, daß** der zweite Flächenanteil (8) am geschlossenen Ende des Schlitzes (6) eine das verdickte Ende (15) wenigstens teilweise aufnehmende Vertiefung aufweist.

4. Prothese nach einem der Ansprüche 1 bis 3, **dadurch gekennzeichnet, daß** das verdickte Ende (15) dem Schlitz (6) eine Konusfläche (16) zukehrt.

## Claims

1. A joint endoprosthesis with an extension part (2) which can be applied or removed along a guide (3,4) provided on the endoprosthesis, and having a screw (5) for locating the extension part (2), which screw has a head (10) and a shank with a thread (12) adjacent the head (10) and which is arranged in the vicinity of surface portions (7,8) mutually overlapping on the prosthesis and on the extension part (2), a first surface portion (7) of which contains a threaded bore (13) accommodating the screw (5), **characterised in that** adjacent the threaded portion (12) the screw shank has a portion (14) of reduced thickness and adjacent thereto has a thickened end (15), wherein the portion (14) of reduced thickness is situated in the vicinity of the second surface portion (8) which has a slot (6) extending in the direction of the guide (3,4) and accommodating the portion (14) of reduced thickness, the width of which slot is smaller than the thickness of the thickened end (15).

2. A prosthesis according to Claim 1, **characterised in that** the first surface portion (7) containing the threaded bore (13) is the more easily accessible.

3. A prosthesis according to Claim 1 or 2,
**characterised in that** at the closed end of the slot (6) the second surface portion (8) has an indentation which at least partly accommodates the thickened end (15).

4. A prosthesis according to any one of Claims 1 to 3,
**characterised in that** the thickened end (15) faces the slot (6) with a conical surface (16).

## Revendications

1. Endoprothèse d'articulation comportant une pièce de liaison (2) qui peut être placée le long d'un guide (3, 4) prévu sur l'endoprothèse, ou enlevée de celui-ci, ainsi qu'une vis (5) pour bloquer la pièce de liaison (2), laquelle présente une tête (10) ainsi qu'une tige avec un filetage (12) voisin de la tête (10) et qui est disposée dans la zone de parties de surface (7, 8) se recouvrant l'une l'autre sur la prothèse et sur la pièce de liaison (2), dont une première partie de surface (7) contient un trou taraudé (13) recevant la vis (5), **caractérisée en ce que** la tige de la vis présente, à la suite de la portion filetée (12), une portion (14) d'épaisseur réduite et, à la suite de celle-ci, une extrémité (15) surépaissie, la portion (14) d'épaisseur réduite se situant dans la zone de la deuxième partie de surface (8) qui présente une fente (6) s'étendant dans la direction du guide (3, 4) et recevant la portion (14) d'épaisseur réduite, fente dont la largeur est inférieure à l'épaisseur de l'extrémité (15) surépaissie.

2. Prothèse selon la revendication 1, **caractérisée en ce que** la première partie de surface (7), qui contient le trou taraudé (13), est celle qui est le plus facilement accessible.

3. Prothèse selon la revendication 1 ou 2, **caractérisée en ce que** la deuxième partie de surface (8) à l'extrémité fermée de la fente (6) présente un renfoncement qui reçoit au moins partiellement l'extrémité (15) surépaissie.

4. Prothèse selon l'une des revendications 1 à 3, **caractérisée en ce que** l'extrémité (15) surépaissie tourne vers la fente (6) une surface de cône (16).
